# FASCICULE DE BREVET EUROPEEN

(11) **EP 2 452 577 B1**
(45) Date de publication et mention de la délivrance du brevet: **20.02.2013**
(21) Numéro de dépôt: 11306480.2
(22) Date de dépôt: 14.11.2011
(51) Int. Cl.: A23L 1/30, A61K 31/6615, A61K 9/08, A61P 39/04

(54) **Solution aqueuse de phytine et utilisation de ladite solution pour la préparation d'un complément alimentaire liquide ou d'une composition cosmétique**
Wässrige Phytinlösung und Verwendung dieser Lösung zur Herstellung eines flüssigen Nahrungsergänzungsmittels oder einer kosmetischen Zusammensetzung
Aqueous solution of phytin and use of said solution for preparing a liquid food supplement or a cosmetic composition

(30) Priorité: 15.11.2010 FR 1059379
(43) Date de publication de la demande: 16.05.2012
(73) Titulaire: Parc, Guy, 92500 Rueil Malmaison (FR)
(72) Inventeur: Parc, Guy, 92500 Rueil Malmaison (FR)
(74) Mandataire: Orsini, Fabienne

(56) Documents cités:
- JP-A- 2008 054 503
- US-A- 2 718 523
- US-A- 2 815 360
- US-A- 3 007 818
- US-A- 3 016 398

## Description

### Domaine de l'invention

La présente invention se rapporte au domaine de la formulation des solutions aqueuses de phytine et à leurs utilisations dans les domaines pharmaceutiques, cosmétiques et alimentaires.

### Etat de la technique

L'acide phytique (CAS : 83-86-3), également appelé acide myo-inositol hexaphosphorique, est une biomolécule présente dans les fruits oléagineux et dans les graines de nombreuses céréales et légumineuses où il représente jusqu'à 90% des réserves totales en phosphore. L'acide phytique se retrouve principalement dans la couche à aleurone ou au niveau de l'embryon des graines où il existe soit sous forme d'acide libre, soit sous forme de phytates associés à différents cations.

L'acide phytique peut être extrait des matrices végétales sous forme de phytine. Le terme « phytine » désigne les sels d'acide de phytique (ou phytates) qui comprennent essentiellement en tant que contre-ions Mg²⁺, Ca²⁺ et/ou K⁺.

L'acide phytique a été longtemps considéré comme un facteur antinutritionnel en alimentation humaine car il limite l'absorption de certains minéraux essentiels tels que le calcium, le magnésium et le fer au niveau du tractus gastro-intestinal.

Néanmoins, des études récentes ont montré que l'acide phytique et la phytine peuvent exercer une large palette d'effets bénéfiques sur la santé humaine (Pour revue : Bohn et al., Journal of Zhejang university Science B, 2008, 9(3) :165-191).

L'acide phytique et la phytine peuvent ainsi participer à l'abaissement du taux de cholestérol, à la normalisation de la glycémie et à la stimulation du système immunitaire. Ces composés peuvent être également utilisés pour prévenir la lithiase rénale, l'hyperpigmentation de la peau mais aussi le vieillissement prématuré des cellules grâce à leur activité antioxydante.

Il a été également montré que l'acide phytique est capable d'inhiber les principaux processus impliqués dans la cancérogénèse tels que la prolifération anormale des cellules et l'angiogénèse. L'acide phytique peut également induire l'apoptose et participer à la stimulation de la réponse immunitaire cellulaire. En conséquence, l'acide phytique et la phytine sont considérés comme des agents particulièrement intéressants pour le traitement mais également pour la prévention du cancer.

Il apparaît donc qu'un apport alimentaire régulier et contrôlé d'acide phytique ou de phytine est susceptible de prévenir l'apparition d'un grand nombre de pathologies.

Dans les pays industrialisés, l'apport moyen en acide phytique fourni par l'alimentation est compris entre 300 à 1200 mg par jour en fonction du régime alimentaire, alors que l'apport quotidien souhaitable pour un équilibre alimentaire convenable est de 2500 mg par jour.

Afin de profiter pleinement des effets bénéfiques de l'acide phytique et de la phytine sur la santé, il est nécessaire de compléter l'apport en acide phytique ou en phytine provenant de l'alimentation par l'intermédiaire de compléments alimentaires dont la prise doit être, de préférence, régulière.

Les compléments alimentaires enrichis en phytine actuellement commercialisés sont composés de gélules ou de comprimés comprenant de la phytine sous la forme d'une poudre insoluble dans l'eau. Ce type de formulation est particulièrement inadapté aux personnes présentant des difficultés à déglutir et également aux personnes âgées et aux jeunes enfants à cause des risques d'étouffement et de fausse route.

Il est possible d'obtenir des solutions aqueuses d'acide phytique. Néanmoins, l'acide phytique présente une acidité élevée et une très faible stabilité en solution aqueuse, ce qui rend son utilisation inadaptée dans un complément alimentaire.

A titre d'exemple de formulations de phytine on peut citer les documents JP 2008 054 503 et US 2 718 523.

Il existe donc, à l'heure actuelle, un besoin pour de nouvelles formulations liquides de phytine qui soient adaptées, entre autres, à la préparation de compléments alimentaires destinés à un large panel de consommateurs.

### Résumé de l'invention

La présente invention a pour objet une solution aqueuse de phytine caractérisée en ce que :
■ elle comprend au moins un acide carboxylique alpha-hydroxylé,
■ elle comprend de la phytine sous forme solubilisée, de préférence à une teneur allant de 70 g/L à 270 g/L et
■ elle a un pH allant de 3 à 5

La présente invention a également pour objet un procédé de préparation d'un complément alimentaire liquide comprenant de la phytine caractérisé en ce qu'il comprend une étape (i) consistant à combiner (a) une phytine, (b) au moins un acide carboxylique alpha-hydroxylé et (c) une solution aqueuse de manière à obtenir une solution aqueuse de phytine solubilisée ayant un pH allant de 3 à 5.

La présente invention a également pour objet un complément alimentaire à base de phytine caractérisé en ce que
(i) il consiste en une solution aqueuse ayant un pH allant de 3 à 7,5 de préférence de 3 à 5 et
(ii) il comprend :
   ■ de la phytine sous forme solubilisée en présence d'au moins un acide carboxylique alpha-hydroxylé, ladite phytine étant présente à une teneur allant de 70 g/L à 270 g/L, et
   ■ en option, au moins un composé supplémentaire choisi parmi le groupe constitué par les minéraux, les vitamines et leurs précurseurs, les acides aminés et leurs dérivés, les oligopeptides, les acides gras, les hormones, les flavonoïdes, les extraits de plantes, le myo-inositol et leurs mélanges.

### Description détaillée de l'invention

### 1. Solution aqueuse de phytine selon la présente invention

Le Demandeur a montré qu'il est possible de solubiliser la phytine en milieu aqueux en la combinant avec au moins un acide carboxylique alpha-hydroxylé. L'acide carboxylique alpha-hydroxylé agit à la fois en tant qu'agent solubilisant et agent stabilisateur de la phytine. L'acide carboxylique alpha-hydroxylé empêche, au moins partiellement, l'hydrolyse des groupements phosphates de la phytine. Le Demandeur a, entre outre, observé que les solutions de phytine solubilisées en présence d'acide carboxylique alpha-hydroxylé présentent une plus grande stabilité que les solutions aqueuses classiques d'acide phytique qui doivent être impérativement stockées à basse température pour limiter les réactions d'hydrolyse.

Sans être lié par une quelconque théorie, le Demandeur est d'avis que les propriétés de stabilisation et de solubilisation des acides carboxyliques alpha-hydroxylés vis-à-vis de la phytine s'expliquent par leur caractère d'acide faible et également par leur propriété de chélation qui résulte, à la fois, des groupements fonctionnels hydroxyle et carboxyle.

De manière surprenante, le Demandeur a montré que les acides carboxyliques alpha-hydroxylés sont plus efficaces que les acides carboxyliques non alpha-hydroxylés pour promouvoir à la fois la solubilisation et la stabilisation de la phytine en solution aqueuse et limiter significativement l'hydrolyse des groupements phosphate.

Le Demandeur pense que l'association de la phytine avec un acide carboxylique alpha-hydroxylé en solution aqueuse génère la formation d'entités supramoléculaires solubles et très stables résultant de liaisons ioniques et de liaisons hydrogènes entre les groupements phosphates des phytates, les cations divalents présents initialement dans la phytine et les groupes fonctionnels hydroxyles et carboxyles de l'acide carboxylique alpha-hydroxylé.

En conséquence, la solution obtenue par combinaison de la phytine avec au moins un acide carboxylique alpha-hydroxylé présente une stabilité élevée dans le temps. Par ailleurs, cette solution est caractérisée par un pH compris entre 3 et 5 ce qui la rend adaptée, entre autres, à une utilisation dans des produits cosmétiques et des complémentaires alimentaires.

Ainsi, un premier objet de l'invention est une solution aqueuse de phytine caractérisée en ce que :
■ elle comprend au moins un acide carboxylique alpha-hydroxylé,
■ elle comprend de la phytine sous forme solubilisée et
■ elle a un pH allant de 3 à 5

Du fait que la phytine est sous forme solubilisée, la solution aqueuse selon la présente invention est limpide. On entend par « solution limpide » une solution pour laquelle aucun trouble ou précipité n'est visible à l'oeil nu.

Au sens de la présente invention, on entend par « *phytine* » les sels d'acide phytique (autremant dit les phytates) comprenant essentiellement en tant que contre-ions Ca²⁺, Mg²⁺ et K⁺. En d'autres termes, la phytine correspond aux formes basiques de l'acide phytique associées principalement aux cations Ca²⁺ et/ou Mg²⁺ et/ou K⁺. La phytine peut comprendre d'autres contre-ions tels que Na⁺, Fe²⁺, Fe³⁺, Co²⁺, Zn²⁺ et Cu²⁺.

La phytine peut être obtenue à partir de graines de céréales. On peut citer comme source végétale adaptée à la préparation d'une phytine selon l'invention le blé, le maïs et le riz. A titre d'exemple, la phytine peut être obtenue à partir de son de blé, de son de riz et de l'embryon des graines de maïs.

Dans certains modes de réalisation, on utilise une phytine obtenue à partir de graines de céréales et présentant un pourcentage massique en calcium de 0,05% à 20% et un pourcentage massique en magnésium de 3% à 18%, les pourcentages étant exprimés par rapport au poids total de phytine.

On entend par « solution aqueuse » une solution comprenant en tant que solvant principal l'eau c'est-à-dire une solution comprenant au moins 90% en volume d'eau, par rapport au volume total de ladite solution. Cette solution aqueuse peut comprendre de 0,1% à 10% en volume d'un solvant miscible dans l'eau. On peut citer comme solvant miscible à l'eau et adapté à la préparation d'un complément alimentaire l'éthanol.

Dans certains modes de réalisation, la solution aqueuse comprend uniquement à titre de solvant de l'eau.

Un pH allant de 3 à 5 englobe un pH de 3, un pH de 3,5, un pH de 4, un pH de 4,5 et un pH de 5.

Au sens de la présente invention, « au moins un » signifie un ou plusieurs. A titre d'exemple, « au moins un acide carboxylique alpha-hydroxylé » englobe 1, 2, 3, 4, 5, 6, 7, 8, 9, 10 acides carboxyliques alpha hydroxylés.

On entend par « acide carboxylique alpha-hydroxylé » une molécule chimique comprenant au moins un groupe fonctionnel acide carboxylique et au moins un groupe fonctionnel hydroxyle situé sur le carbone en alpha dudit groupe fonctionnel acide carboxylique.

De préférence, les acides carboxyliques alpha-hydroxylés correspondent à la la formule (I) suivante : 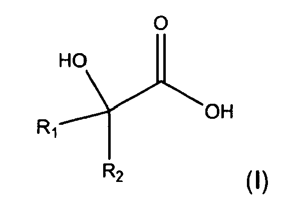 dans laquelle :
- R1 et R2 sont indépendamment choisis parmi l'hydrogène et les groupes alkyles en C₁-C₁₄ linéaires ou ramifiés, non substitués ou substitués par un ou plusieurs groupes choisis parmi le groupe acide carboxylique et le groupe hydroxyle, ou
- R1 et R2 forment un groupe cyclique choisi parmi les groupes cycloalkyles en C₅-C₈ et les hétérocyclalkyles en C₄-C₇, ledit groupe cyclique étant non substitué ou substitué par un ou plusieurs groupes choisis indépendamment parmi le groupe constitué par le groupe acide carboxylique et le groupe hydroxyle.

Au sens de l'invention, un groupe cycloalkyle comprend, entre autres, le cyclopentyle, le cyclohexyle et le cycloheptyle.

Au sens de l'invention, l'hétérocycloalkyle en C₄-C₇ comprend en tant qu'hétéroatome un atome d'oxygène ou un atome d'azote. On peut citer à titre d'exemple d'acide carboxylique alpha-hydroxylé hétérocyclique l'acide glucuronique.

Dans certains modes de réalisation, l'acide carboxylique alpha-hydroxylé est choisi parmi les composés de formule 1 dans laquelle :
- R1 est un hydrogène et
- R2 est un groupe alkyle en C₁-C₁₀, linéaire ou ramifié, non substitué ou substitué par un ou plusieurs groupes choisis indépendamment parmi le groupe constitué par le groupe acide carboxylique et le groupe hydroxyle.

Un groupe alkyle en C₁-C₁₀ comprend les groupes alkyles en C₁, en C₂, en C₃, en C₄, en C₅, en C₆, en C₇, en C₈, C₉ et en C₁₀.

De manière préférée, R2 est un groupe alkyle en C₁-C₅. Les radicaux alkyles en C₁-C₅ comprennent les radicaux méthyle, éthyle, propyle, butyle et pentyle.

Il va de soi que l'acide carboxylique alpha-hydroxylé à utiliser est choisi en fonction de l'utilisation prévue de la solution de phytine selon la présente invention. A titre d'exemple, si la solution de phytine selon l'invention est destinée à être utilisée dans la préparation d'un complément alimentaire ou d'un produit cosmétique, l'acide carboxylique alpha-hydroxylé doit présenter une faible toxicité et, si possible, un faible pouvoir allergisant.

Dans certains modes de réalisation, l'acide carboxylique alpha-hydroxylé comprend au moins un groupe acide carboxylique ayant un pKa compris dans une gamme allant de 2,9 à 4,0.

L'acide carboxylique alpha hydroxylé peut être choisi parmi les additifs alimentaires, les excipients utilisables pour la préparation des compléments alimentaires, des compositions pharmaceutiques ou des compositions cosmétiques.

Dans un mode de réalisation préféré, l'acide carboxylique alpha-hydroxylé est choisi parmi le groupe constitué par l'acide glycolique, l'acide gluconique, l'acide glucuronique, les diastéréoisomères de l'acide tartrique, l'acide lactique, l'acide citrique, l'acide malique et leurs mélanges.

Les diastéréoisomères de l'acide tartrique englobent l'acide tartrique méso, l'acide tartrique lévogyre et l'acide tartrique dextrogyre.

Le Demandeur a montré que les acides carboxyliques alpha-hydroxylés permettent d'augmenter de manière significative la solubilité de la phytine dans l'eau. Ainsi, il est donc possible d'obtenir des solutions comprenant une teneur élevée de phytine à l'état solubilisé. Dans un mode de réalisation préféré, la teneur en phytine est comprise dans une gamme allant de 70 g/L à 270 g/L, la phytine étant à l'état solubilisé et étant entendu que la teneur en phytine est exprimée par rapport au volume total de la solution de phytine.

Une teneur en phytine allant de 70 g/L à 270 g/L englobe une teneur en phytine de 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 210 g/L, 220 g/L, 230 g/L, 240 g/L, 250 g/L, 260 g/L et 270 g/L.

Pour obtenir une solubilisation et une stabilisation optimale de phytine, le Demandeur a montré qu'il convient de fournir une quantité d'acide carboxylique alpha-hydroxylé correspondant à un apport en groupe fonctionnel carboxyle allant de 3 mmol à 9 mmol par gramme de phytine. La quantité d'acide carboxylique alpha-hydroxylé à apporter pour obtenir une solution stable de phytine varie donc en fonction du nombre de groupements carboxyles présents sur ledit acide carboxylique alpha-hydroxylé. Ainsi, un apport en groupement fonctionnel carboxyle de 3 mmol à 9 mmol par gramme de phytine correspond, par exemple à :
■ une quantité d'acide carboxylique alpha-hydroxylé ayant un seul groupement carboxyle allant de 3,0 mmol à 9,0 mmol par gramme de phytine,
■ une quantité d'acide carboxylique alpha-hydroxylé ayant deux groupements carboxyles allant de 1,5 mmol à 4,5 mmol par gramme de phytine,
■ une quantité d'acide carboxylique alpha-hydroxylé ayant trois groupements carboxyles allant de 1,0 mmol à 3,0 mmol par gramme de phytine, ou encore à
■ une quantité d'acide carboxylique alpha-hydroxylé ayant quatre groupements carboxyles allant de 0,75 mmol à 2,25 mmol par gramme de phytine

Ainsi, dans certains modes de réalisation, la quantité d'acide carboxylique alpha-hydroxylé qui est combiné à la phytine dans la solution de phytine selon la présente invention correspond à un apport en groupe fonctionnel carboxyle allant de 3 mmol à 9 mmol par gramme de phytine.

Un apport en groupe fonctionnel carboxyle allant de 3 mmol à 9 mmol englobe un apport en groupe fonctionnel carboxyle de 3 mmol, 3,5 mmol, 4 mmol, 4,5 mmol, 5 mmol, 5,5 mmol, 6 mmol, 6,5 mmol, 7 mmol, 7,5 mmol, 8 mmol, 8,5 mmol et 9 mmol.

De préférence, l'apport en groupe fonctionnel carboxyle est compris dans une gamme allant de 4.5 mmol à 8 mmol par gramme de phytine.

A partir de ces données et en fonction de la teneur finale en phytine solubilisée que doit contenir la solution de phytine selon la présente invention, l'homme du métier sait déterminer par des expériences de routine la quantité exacte de groupe fonctionnel carboxyle à fournir par gramme de phytine et donc par voie de conséquence, la quantité d'acide carboxylique alpha hydroxylé à utiliser par gramme de phytine.

Par exemple, une solution ayant une teneur de 140 g/L de phytine peut être obtenue en fournissant une quantité d'acide citrique d'environ 2 mmol par gramme de phytine, ce qui correspond à un apport en groupe fonctionnel carboxyle d'environ 6 mmol par gramme de phytine.

La solution de phytine selon la présente invention peut être utilisée dans différents types d'applications, en particulier dans les domaines pharmaceutique, alimentaire et cosmétique.

A titre d'exemple, la solution de phytine selon la présente invention peut être incorporée dans des compositions cosmétiques telles que des crèmes, des solutions lavantes, des lotions et des produits d'hygiène dentaire.

Ainsi, un objet spécifique de l'invention est l'utilisation d'une solution aqueuse de phytine, ladite phytine ayant été solubilisée en présence d'au moins un acide carboxylique alpha-hydroxylé, pour la préparation d'une composition cosmétique.

Lorsqu'elle est incorporée dans des crèmes ou des lotions, la solution de phytine peut jouer le rôle d'agent éclaircissant, d'agent prévenant l'hyperpigmentation, de substitut de l'EDTA, d'agent anti-ride, d'agent anti-vieillissement ou encore d'agent antioxydant.

La solution de phytine selon l'invention peut être incorporée également dans des dentifrices ou des lotions dentaires en raison de son action sur la formation de la plaque dentaire et sur l'émail dentaire.

La solution de phytine peut représenter de 0,2% à 95% en poids du poids total de la composition cosmétique considérée. Pour la préparation de ces compositions cosmétiques, l'homme du métier pourra se référer aux ouvrages de référence du domaine, en particulier à l'ouvrage « International Cosmetic Ingredient Dictionary and Handbook, 8ème édition, 2000 publié par The Cosmetic, Toiletry and Fragrance Association.

La solution de phytine selon la présente invention peut être avantageusement utilisée pour la préparation de compléments alimentaires.

Cette utilisation spécifique est présentée en détails dans ce qui suit.

### 2. Complément alimentaire liquide et procédé de préparation dudit complément alimentaire.

### a. Procédé de préparation d'un complément alimentaire liquide selon l'invention

Sans être lié par une quelconque théorie, le Demandeur est d'avis que les solutions de phytine selon la présente invention présentent une biodisponibilité en phytine plus élevée que les formulations solides de phytine décrites dans l'état de la technique. En conséquence, la solution aqueuse de phytine selon la présente invention est particulièrement adaptée à une utilisation en tant que complément alimentaire ou à une utilisation pour la préparation d'un complément alimentaire.

Ainsi, un second objet de la présente invention est un procédé de préparation d'un complément alimentaire liquide comprenant de la phytine caractérisé en ce qu'il comprend une étape (i) consistant à préparer une solution de phytine telle que décrite de manière approfondie dans la première partie la présente description intitulée « Solution aqueuse de phytine selon la présente invention ».

En d'autres termes, le procédé de préparation du complément alimentaire liquide comprenant de la phytine est caractérisé en ce qu'il comprend une étape (i) consistant à combiner (a) une phytine, (b) au moins un acide carboxylique alpha-hydroxylé et (c) un solvant aqueux de manière à obtenir une solution aqueuse ayant un pH allant de 3 à 5 et comprenant de la phytine dans un état solubilisé.

Des modes de réalisation du procédé selon la présente invention relatifs aux caractéristiques de l'étape (i) sont déductibles directement des éléments décrits dans la partie 1 intitulée « Solution aqueuse de phytine selon la présente invention ».

En particulier, la teneur finale en phytine dans le complément alimentaire est de préférence comprise dans une gamme allant de 70 g/L à 270 g/L, la phytine étant à l'état solubilisé et étant entendu que la teneur en phytine est exprimée par rapport au volume total du complément alimentaire.

De préférence, l'acide carboxylique alpha-hydroxylé utilisé à l'étape (i) vérifie la formule chimique (I) ci-dessus. Comme expliqué précédemment, pour obtenir une solubilisation et une stabilisation optimale de la phytine, il convient de fournir à l'étape (i) une quantité d'acide carboxylique alpha-hydroxylé correspondant à un apport en groupe fonctionnel carboxyle allant de 3 mmol à 9 mmol par gramme de phytine.

On entend par « solvant aqueux » un liquide comprenant majoritairement de l'eau, c'est-à-dire comprenant au moins 90% d'eau en volume. Un solvant aqueux peut donc comprendre de 0,1% à 10% en volume d'un solvant miscible dans l'eau. On peut citer comme solvant miscible à l'eau et adapté à la préparation d'un complément alimentaire l'éthanol.

Dans certains modes de réalisation, le solvant aqueux consiste en de l'eau.

Dans des modes de réalisation supplémentaires, l'étape (i) du procédé selon la présente invention comprend la séquence d'étapes suivantes consistant à :
(a) préparer une suspension de phytine dans une solution aqueuse
(b) ajouter, sous agitation, à la suspension obtenue à l'étape (a), une quantité d'au moins un acide carboxylique alpha-hydroxylé correspondant à un apport en groupe fonctionnel carboxyle allant de 3 mmol à 9 mmol par gramme de phytine, et
(c) maintenir sous agitation la suspension de phytine additionnée de l'acide carboxylique alpha-hydroxylé obtenue à l'étape (b) jusqu'à l'obtention d'une solution limpide.

On entend par « une solution limpide » une solution pour laquelle aucun trouble ou précipité n'est visible à l'oeil nu.

Il est préférable dans l'étape (b) d'ajouter l'acide carboxylique alpha-hydroxylé progressivement ou par fractions et de mettre le milieu réactionnel sous agitation de manière à éviter la prise en masse.

L'acide carboxylique alpha-hydroxylé peut être introduit sous la forme d'une solution concentrée. On entend par une solution concentrée en acide carboxylique alpha-hydroxylée une solution possédant une concentration au moins égale à 0,3 mol.l⁻¹. Afin d'obtenir une solubilisation totale de la phytine, il peut être nécessaire de maintenir le mélange de l'étape (b) sous agitation. La réaction étant exothermique, il n'est pas nécessaire, en général, de chauffer le milieu réactionnel pour atteindre une solubilisation complète de la phytine.

Le complément alimentaire obtenu par le procédé selon l'invention peut comprendre au moins un composé supplémentaire présentant un intérêt nutritionnel, physiologique ou pharmaceutique. Il peut s'agir d'un composé pouvant agir en combinaison avec la phytine, par exemple, un composé capable de stimuler les défenses immunitaires ou encore d'un composé ayant une action distincte de celles de la phytine. De manière avantageuse, le composé supplémentaire peut être un nutriment essentiel pour l'organisme humain, c'est-à-dire un nutriment que le corps humain n'est pas capable de synthétiser.

Ainsi, dans certains modes de réalisation, le procédé selon l'invention comprend l'étape (ii) consistant à ajouter à la solution obtenue à l'étape (i) au moins un composé supplémentaire choisi parmi le groupe constitué par les minéraux, les vitamines et leurs précurseurs, les acides aminés, les dérivés des acides aminés, les oligopeptides, les acides gras, les hormones, les flavonoïdesle myo-inositol, les extraits de plantes et leurs mélanges.

Au sens de l'invention, les minéraux comprennent l'ensemble des substances inorganiques décrites dans l'état de la technique comme essentielles au bon fonctionnement du corps humain. Les minéraux comprennent le calcium, le magnésium, l'iode, le fer, le cuivre, le zinc, le sélénium et manganèse, la présente liste n'étant pas exhaustive.

Les vitamines comprennent des vitamines hydrosolubles telles que les vitamines B, la vitamine C et les vitamines liposolubles telles que les vitamines A, D, E et K.

En général, le terme « vitamine » regroupe un ensemble de composés. Dans ce qui suit, on entend ainsi par vitamine E l'ensemble des formes naturelles de la vitamine E c'est-à-dire l'alpha-tocophérol, le bêta-tocophérol, le gamma-tocophérol, le delta-tocophérol, l'alpha-tocotriénol, le bêta-tocotriénol, le gamma-tocotriénol et le delta-tocotriénol. De la même manière, lorsque l'on parle de vitamine D, on se réfère indifféremment à l'ergocalciférol et au cholécalciférol.

On entend par précurseur des vitamines les composés participant au cycle de biosynthèse des vitamines. A titre d'exemple, le 7-déhydrocholestérol est un précurseur de la vitamine D.

Au sens de l'invention, on entend par « acide aminé » une molécule chimique comprenant une fonction amine primaire positionnée en alpha d'un groupe acide carboxylique. Le terme « acide aminé » englobe les acides aminés naturels, en particulier les acides aminés du code génétique, et les acides aminés de synthèse. De manière préférée, l'acide aminé est choisi parmi les acides aminés basiques tels que L-histidine, la L-ornithine, la L-lysine et la L-arginine

Au sens de l'invention, les oligopeptides comprennent de 2 à 50 acides aminés, de préférence de 2 à 10 acides aminés. De manière préférée, l'oligopeptide comprend au moins un acide aminé choisi parmi la L-histidine, la L-ornithine, la L-lysine et la L-arginine.

On peut citer à titre d'exemple de dérivés d'acides aminés la taurine.

Les acides gras comprennent, en particulier, la famille des oméga 3. A titre illustratif et non limitatif, il peut être cité en tant qu'acides gras appartenant à la famille des omégas 3, l'acide α-linolénique, l'acide éicosapentaénoïque et l'acide docosahexaénoïque.

Les flavonoïdes regroupent des biomolécules végétales comprenant un motif central flavone. On peut citer à titre d'exemple la quercétine et l'hespéridine.

Le myo-inositol correspond à la forme déphosphorylée de l'acide phytique.et correspond au composé de numéro de CAS : 87-89-8.

On peut citer, à titre d'exemple, d'hormones la mélatonine, la DHEA et les phyto-oestrogènes.

Enfin, on peut citer, à titre d'exemple d'extraits de plantes, les extraits de ginseng, de rhodiola, de guarana et de kola.

Le ou les composés supplémentaires à ajouter à l'étape (ii) peuvent être déterminés en fonction des effets physiologiques recherchés ou en fonction du public visé. A titre d'exemple, si on recherche de fournir une activité antioxydante élevée, on pourra associer à la phytine, par exemple, un composé choisi parmi la vitamine C, la vitamine E ou la quercétine ou encore un extrait de thé vert. Si le complément alimentaire est destiné à un consommateur souffrant d'un déséquilibre alimentaire, on pourra combiner l'acide phytique avec un cocktail de minéraux et de vitamines. Si l'effet physiologique recherché est de renforcer la résistance au stress, ou d'améliorer la concentration intellectuelle ou l'endurance physique, le complément alimentaire selon la présente invention peut comprendre un extrait de plantes tel qu'un extrait de ginseng (*Panax ginseng C.A.Meyer*), un extrait de rhodiola (*Rhodiola rosea L.*), de guarana (*Paullinia cupana Kunth*) et de kola (*Cola nitida alba, Cola nitida rubra A. Chev,* ou encore *Cola acuminata Schott & Endl*)).

Le ou les composés supplémentaires à but physiologique, pharmaceutique ou nutritionnel sont généralement présents dans le complément alimentaire liquide à une teneur allant de 0,01 g/L à 900 g/L, de préférence, de 0,1 g/L à 400 g/L. La teneur d'un composé supplémentaire est à déterminer en fonction de la nature de ce composé et de l'effet recherché. Les composés supplémentaires étant couramment utilisés dans la préparation des compléments alimentaires et des compositions pharmaceutiques, l'homme du métier peut se référer aux doses usuelles utilisées dans l'état de la technique pour déterminer, pour chaque composé supplémentaire, la concentration à laquelle il doit être introduit dans le complément alimentaire selon la présente invention.

Dans un mode de réalisation préféré selon l'invention, on rajoute à l'étape (ii) du procédé du myo-inositol et un composé de la vitamine C c'est-à-dire de l'acide ascorbique ou l'un de ses sels.

Sans être lié par une quelconque théorie, le Demandeur est d'avis qu'une telle combinaison est particulièrement adaptée pour la prévention des cancers et la stimulation du système immunitaire. En effet, il a été montré que l'association de la phytine avec le myo-inositol est capable de moduler certains processus biologiques impliqués dans la cancérogénèse tels que la prolifération anormale des cellules et l'angiogénèse.

Par ailleurs, il est attendu que l'acide phytique et la vitamine C agissent en synergie pour stimuler la réponse immunitaire et prévenir le stress oxydant et donc le vieillissement cellulaire.

De manière préférée, le myo-inositol est introduit à l'étape (ii) du procédé selon l'invention de manière à ce que sa teneur finale dans le complément alimentaire soit comprise dans une gamme allant de 3 g/L à 6 g/L. L'acide ascorbique ou l'un de ses sels est introduit de manière à ce que sa teneur finale dans le complément alimentaire soit comprise dans une gamme allant de 6g/L à 12 g/L.

Comme présenté ci-dessus, le procédé de préparation d'un complément alimentaire liquide comprenant une teneur en phytine allant de 70 g/L à 270 g/L selon la présente invention comprend les étapes consistant à :
(i) combiner une phytine, au moins un acide carboxylique alpha-hydroxylé et une solution aqueuse de manière à obtenir une solution aqueuse de phytine ayant un pH allant de 3 à 5 et
(ii) optionnellement, ajouter à la solution obtenue à l'étape (i) au moins un composé supplémentaire choisi parmi le groupe constitué par les minéraux, les vitamines et leurs précurseurs, les acides aminés et leurs dérivés, les oligopeptides, les extraits de plantes, les acides gras, les hormones, les flavonoïdes, le myo-inositol, et leurs mélanges. Le procédé selon la présente invention peut comprendre de plus, une étape de filtration de la solution obtenue à l'étape (i) et/ou à l'étape (ii). Cette étape de filtration permet d'éliminer les éventuelles particules solides résiduelles en suspension.

Enfin, le procédé selon la présente invention peut comprendre une étape finale de conditionnement. A titre d'exemple, le complément alimentaire selon l'invention peut être conditionné dans un contenant multi-doses ou dans des contenants unidoses.

Le procédé de préparation selon la présente invention peut être mis en oeuvre par des méthodes couramment utilisées dans l'industrie alimentaire et l'industrie pharmaceutique. En particulier, il est nécessaire de mettre en oeuvre le procédé de préparation selon la présente invention en respectant les Bonnes Pratiques de Laboratoires et les normes réglementaires en vigueur. Le procédé de préparation selon la présente invention doit être réalisé dans des conditions aseptiques avec des matières premières présentant une pureté adaptée à leur utilisation dans un complément alimentaire.

### b.Complément alimentaire liquide enrichi en phytine selon la présente invention

La présente invention a également pour objet un complément alimentaire liquide comprenant de la phytine pouvant être obtenu par le procédé de préparation décrit ci-dessous.

Le complément alimentaire liquide selon la présente invention est caractérisé en ce que :
(i) il s'agit d'une solution aqueuse ayant un pH allant de 3 à 7,5, de préférence de 3 à 5, et
(ii) il comprend de la phytine sous forme solubilisée en présence d'au moins un acide carboxylique alpha-hydroxylé, ladite phytine étant présente à une teneur allant de 70 g/L à 270 g/L, étant entendu que ladite teneur en phytine est exprimée par rapport au volume total dudit complément alimentaire.

Comme expliqué précédemment, on entend par solution aqueuse, une solution comprenant l'eau comme solvant majoritaire.

Dans certains modes de réalisation, le complément alimentaire selon la présente invention peut contenir une faible proportion d'un solvant miscible à l'eau, par exemple de 0,5% à 10% en volume d'un solvant tel que l'éthanol, ce pourcentage étant exprimé par rapport au volume total dudit complément alimentaire. Le pourcentage volumique d'eau dans le complément alimentaire est alors compris dans la gamme allant de 99,5% à 90%.

Dans d'autres modes de réalisation, l'eau est le seul solvant présent dans le complément alimentaire selon la présente invention.

Comme décrit précédemment, pour permettre une solubilisation et une stabilisation optimale de la phytine, la quantité d'acide carboxylique alpha-hydroxylé présent en solution doit de préférence correspondre à une quantité de groupe fonctionnel carboxyle allant de 3 mmol à 9 mmol par gramme de phytine.

Dans un mode de réalisation préféré, l'acide carboxylique alpha-hydroxylé est choisi parmi l'acide citrique, l'acide lactique, l'acide tartrique, l'acide malique et leurs mélanges.

Le complément alimentaire liquide peut comprendre au moins un composé supplémentaire à but nutritionnel, physiologique ou pharmaceutique.

Ainsi, le complément alimentaire liquide comprenant de la phytine selon la présente invention est caractérisé en ce que :
(i) il s'agit d'une solution aqueuse ayant un pH allant de 3 à 7,5, de préférence de 3 à 5,
(ii) il comprend de la phytine sous forme solubilisée en présence d'au moins un acide carboxylique alpha-hydroxylé, ladite phytine étant présente à une teneur allant de 70 g/L à 270 g/L, étant entendu que ladite teneur en phytine est exprimée par rapport au volume total dudit complément alimentaire et
(iii) facultativement, au moins un composé supplémentaire choisi parmi le groupe constitué par les minéraux, les vitamines et leurs précurseurs, les acides aminés, les dérivés des acides aminés, les oligopeptides, les extraits de plantes, les acides gras, les hormones, les flavonoïdes, le myo-inositol, et leurs mélanges. Comme expliqué précédemment, le composé supplémentaire peut soit renforcer les effets bénéfiques de la phytine, soit produire un effet physiologique autres que ceux exercés par la phytine

Sur la base de ses connaissances générales en physiologie et en pharmacie, l'homme du métier pourra sélectionner le ou les composés supplémentaires à ajouter en fonction (i) du consommateur visé et (ii) de l'effet physiologique recherché.

Ainsi si on recherche à la fois (i) une action antioxydante, (ii) une action prophylactique vis-à-vis du cancer et (iii) une stimulation du système immunitaire, il est avantageux de formuler un complément alimentaire liquide comprenant à la fois de la phytine, un composé de la vitamine C et du myo-inositol.

Par ailleurs, si l'on cherche à potentialiser l'activité anti-cancéreuse de la phytine, le complément alimentaire peut comprendre, de manière avantageuse, au moins un composé choisi parmi les acides aminés basiques tels que la L-lysine, la L-histidine, la L-arginine et la L-ornithine et les oligopeptides comprenant au moins un acide aminé basique dans leur structure. Par exemple, le complément alimentaire peut comprendre une peptone de soja. Les peptones de soja sont riches en arginine ou lysine à l'état libre ou sous forme d'oligopeptides en fonction de leur degré d'hydrolyse.

Le ou les composés supplémentaires à but physiologique, pharmaceutique ou nutritionnel sont généralement présents dans le complément alimentaire liquide à une teneur allant de 0,01 g/L à 900 g/L, de préférence, de 0,1 g/L à 400 g/L. La teneur d'un composé supplémentaire est à déterminer en fonction de la nature de ce composé et de l'effet recherché. L'homme du métier peut se référer aux doses usuelles utilisées dans l'état de la technique pour déterminer, pour chaque composé supplémentaire, la concentration à laquelle ledit composé doit être introduit dans le complément alimentaire selon la présente invention.

Dans un mode de réalisation particulier, le complément alimentaire selon la présente invention comprend, en outre, du myo-inositol à une teneur allant, de préférence, de 3 g/L et 6 g/L et de l'acide ascorbique ou l'un de ses sels à une teneur allant de 6 g/L à 12 g/L.

Les doses journalières de phytine et des composés à but nutritionnel, physiologique ou pharmaceutique éventuellement présents dans le complément alimentaire peuvent être déterminées en prenant en compte, entre autres, les apports nutritionnels journaliers recommandés dans l'état de la technique. La posologie du complément alimentaire pourra être également ajustée en fonction de l'effet physiologique recherché et en fonction de l'état physiologique du consommateur auquel est destiné le complément alimentaire. En particulier, on pourra prendre en compte des facteurs individuels tels que l'âge, le poids, le sexe, l'état général et le régime alimentaire du consommateur auquel on destine le complément alimentaire liquide.

Concernant la phytine, un apport nutritionnel journalier d'au moins 2500 mg est recommandé alors que le régime alimentaire suivi dans les pays industrialisés apporte de 300 mg à 1200 mg par jour de phytine. Ainsi, la dose journalière en phytine à apporter au consommateur se situe de préférence dans une gamme allant de 1300 mg à 2200 mg.

Le complément alimentaire liquide selon la présente invention peut comprendre, en outre, un ou plusieurs excipients choisis parmi les excipients pharmaceutiques ou les additifs alimentaires.

A titre d'exemple d'excipients pharmaceutiques et d'additifs alimentaires, il peut être cité les agents texturants, les agents émulsifiants, les agents conservateurs, les colorants, les arômes pouvant être naturels ou de synthèse, les édulcorants et les exhausteurs de goût.

Dans certains modes de réalisation, le complément alimentaire liquide selon la présente invention comprend au moins un excipient choisi parmi les agents conservateurs, les colorants, les arômes, les édulcorants et les exhausteurs de goût.

Ces composés peuvent être utilisés afin de rendre plus agréable la prise du complément alimentaire liquide par le consommateur. Ceci est particulièrement important lorsque le consommateur visé est un enfant. A titre d'exemple, afin de faciliter et de rendre plus agréable la prise du complément alimentaire, on peut ajouter au complément alimentaire selon l'invention :
(i) un arôme reproduisant le goût d'agrume
(ii) un colorant apportant une coloration jaune-orangée
(iii) un agent édulcorant
de manière à donner la sensation de jus de fruits lors de la prise dudit complément alimentaire.

Comme exposé précédemment, le complément alimentaire selon la présente invention présente une grande stabilité dans le temps. Il n'est donc pas forcément nécessaire d'ajouter au complément alimentaire un agent conservateur ou stabilisateur lorsqu'il est conditionné par unité de dose.

Ainsi, dans certains modes de réalisation, le complément alimentaire liquide ne comprend pas d'agent conservateur ou stabilisateur autre que l'acide carboxylique alpha-hydroxylé. A titre illustratif, on peut citer en tant qu'agent conservateur le sorbate de sodium, le benzoate de potassium, l'acide acétique, le nitrite de sodium, la liste n'étant pas exhaustive.

Lorsque le complément alimentaire est conditionné en multi-doses, il est préférable d'ajouter un agent conservateur afin d'éviter la contamination bactérienne ou fongique.

Dans d'autres modes de réalisation, le complément alimentaire est exempt de tout agent texturant tels que les agents épaississants et les agents gélifiants. En effet, la présence de ces agents peut être préjudiciable au caractère liquide et à l'homogénéité du complément alimentaire. En particulier, le complément alimentaire ne contient pas de composés gélifiants tels que l'agar-agar, l'amidon, les sels d'alginate, le carraghénane et les gélatines animales.

Le complément alimentaire selon la présente invention peut être conditionné en contenant multi-dose ou en contenant unidose. De manière préférée, le complément alimentaire est conditionné en unités de dose, par exemple sous la forme de dosettes ou d'ampoules unidoses sécables.

Le complément alimentaire liquide selon la présente invention peut être administré pour compléter les apports en phytine issus de l'alimentation. En d'autres termes, le complément alimentaire selon l'invention est, entre autres, destiné à assurer au consommateur un apport journalier régulier et suffisant en phytine. Un tel apport permet de maintenir ou d'améliorer la condition générale du consommateur auquel est administré le complément alimentaire selon l'invention.

Il peut être également prescrit à un consommateur présentant un désordre physiologique ou susceptible de développer un désordre physiologique ou une pathologie. En particulier, le complément alimentaire selon l'invention peut être prescrit pour prévenir la lithiase rénale ou le vieillissement cellulaire, pour normaliser le taux de cholestérol ou le taux de glycémie, stimuler le système immunitaire ou prévenir pour l'apparition du cancer. Le complément alimentaire selon la présente invention peut être également prescrit pour améliorer la résistance au stress, la concentration intellectuelle et l'endurance physique.

Le complément alimentaire selon la présente invention peut être également utilisé à des fins cosmétiques pour maintenir ou améliorer l'état général de la peau en prévenant son vieillissement et l'apparition de taches d'hyperpigmentation.

Il peut être également utilisé afin de renforcer l'action d'un traitement thérapeutique ou d'améliorer l'état général d'un patient subissant un traitement thérapeutique lourd, ayant subi un traitement chirurgical et/ou ne pouvant pas s'alimenter normalement. Il peut être avantageux, en particulier, de prescrire le complément alimentaire selon l'invention à des patients subissant des traitements chimiothérapiques ou radiothérapiques.

Le complément alimentaire liquide à base de phytine selon la présente invention présente plusieurs avantages par rapport aux compléments alimentaires commercialisés qui, à la connaissance du Demandeur, consistent en des gélules ou des comprimés comprenant de la poudre de phytine.

De par sa présentation sous forme liquide, le complément alimentaire peut être administré indifféremment aux enfants, aux personnes âgées et à toute personne présentant des difficultés pour déglutir. Il est attendu que l'observance des prises soit respectée par les consommateurs du fait de sa formulation sous forme liquide.

Enfin, comme évoqué précédemment il est attendu que la phytine présente dans le complément alimentaire selon la présente invention, qui est sous une formes solubilisée en présence d'acide carboxylique alpha-hydroxylé, présente une biodisponibilité plus élevée que la phytine sous forme de gélule ou de comprimé.

Un autre objet de la présente invention est l'utilisation d'une solution aqueuse de phytine, ladite phytine ayant été solubilisée en présence d'au moins un acide carboxylique alpha-hydroxylé, pour la préparation d'un complément alimentaire.

La présente invention est également illustrée par les exemples présentés ci-après, sans y être limitée.

### Exemples

### Exemple 1 :

### a. Solubilisation de la phytine par les acides carboxyliques

Afin de comparer le pouvoir solubilisant de différents acides carboxyliques entre eux, le mode opératoire suivant a été réalisé :
Pour chaque acide carboxylique, une solution aqueuse de normalité 0,3 mol.l⁻¹ a été préparée.

On rappelle que la normalité d'une solution d'acide correspond au nombre de moles de protons H⁺ fournies par 1 litre de cette solution. Ainsi une solution d'acide citrique ayant une normalité de 0,3 N est équivalent à une solution d'acide citrique ayant une molarité de 0,1 mol.l⁻¹ car l'acide citrique comporte 3 groupes acides carboxyliques.

En d'autres termes 1 mole d'acide citrique est susceptible de fournir 3 moles de protons H⁺.

Une suspension de phytine est préparée par suspension de 1 g de phytine dans un volume de 10 ml d'eau distillée. La phytine utilisée est la phytine commercialisée par Tsuno Rice Fine Chemicals Co., Ltd sous la référence PHYTIN (RICE BRAN EXTRACT). Sa composition est présentée dans le tableau 1 ci-après. Il est à noter que cette phytine est riche en magnésium.

**Tableau 1 : Composition de la phytine utilisée**

| Phytine riche en magnésium commercialisée par Tsuno Rice Fine Chemicals Co., Ltd extraite du son de riz (Wakayama, Japon) | |
|---|---|
| Pureté | 99,7% |
| Teneur en phosphore | 20% |
| Teneur en magnésium | 12,7% |
| Teneur en calcium | 0,5% |
| Teneur en potassium | 4,6% |

| | |
|---|---|
| * les teneurs correspondent à des pourcentages massiques par rapport au poids total de la phytine commerciale. | |

La solution d'acide carboxylique à 0,3 N est ajoutée progressivement dans la suspension de phytine sous agitation à l'aide d'une burette graduée selon un protocole équivalent à un titrage pHmétrique. L'évolution du pH est suivi à l'aide d'un pHmètre et le volume de solution d'acide carboxylique nécessaire à la solubilisation totale de la phytine est déterminée.

Le tableau 2 ci-dessous présente les résultats obtenus pour les différents acides carboxyliques testés.

Il apparaît clairement que les acides carboxyliques présentant au moins un hydroxyle en position alpha d'un carboxyle présente un pouvoir solubilisant plus important que les acides carboxyliques non-alpha hydroxylé. En effet la quantité de groupes fonctionnels carboxyles à fournir dans le cas des acides carboxyliques alpha-hydroxylés est bien nettement inférieure à celle des acides carboxyliques non alpha-hydroxylés. Ce fait ressort nettement des résultats obtenus pour l'acide malique et l'acide succinique qui comportent chacun 2 groupes acides carboxyliques.

Le nombre de groupements carboxyles portés par l'acide carboxylique alpha-hydroxylé ne semble pas avoir d'incidence directe sur son pouvoir de solubilisation de la phytine.

**Tableau 2 : Evaluation du pouvoir solubilisant des acides carboxyliques vis-à-vis de la phytine commerciale extraite du son de riz**

| ***Acide carboxylique alpha-hydroxylé*** | | | |
|---|---|---|---|
| Solution d'acide | Nombre de groupes carboxyles | Volume ajouté pour solubiliser totalement la phytine | Quantité de groupes fonctionnels carboxyles apportés (par gramme de phytine) |
| Acide malique à 0,15 mol.l⁻¹ | 2 dont 1 avec hydroxyle en alpha | 17,5 ml | 5,25 mmol |
| Acide citrique à 0,1 mol.l⁻¹ | 3 dont 1 avec hydroxyle en alpha | 17,0 ml | 5,10 mmol |
| Acide tartrique à 0,15 mol.l⁻¹ | 2 groupes dont 2 avec hydroxyle en position alpha | 17,2 ml | 5,16 mmol |

| ***Acide carboxylique non alpha-hydroxylé*** | | | |
|---|---|---|---|
| Solution d'acide | Nombre de groupes carboxyles | Volume ajouté pour solubiliser totalement la phytine | Quantité de groupes fonctionnels carboxyles apportés |
| Acide acétique à 0,3 mol.l⁻¹ | 1 | 35,5 ml | 10,65 mmol |
| Acide succinique à 0,15 mol.l⁻¹ | 2 | 36,0 ml | 10,80 mmol |

### b. Influence de la composition de la phytine

Des expériences selon un protocole identique à celui du point a. ci-dessus ont été réaliséesavec une phytine commerciale riche en calcium également commercialisée par Tsuno Rice Fine Chemicals Co., Ltd (voir tableau 3 ci-après). Deux solutions d'acide carboxylique à 0,3 N ont été testées, à savoir l'acide citrique et l'acide acétique.

L'acide citrique est très efficace pour solubiliser cette forme de phytine caractérisée par une teneur élevée en calcium. En effet, un volume de 22,3 mL d'une solution d'acide citrique à 0,3 N (ce qui correspond à un apport en groupe carboxyle de 6,7 mmol) permet une solubilisation totale de la phytine et l'obtention d'une solution limpide.

En revanche, l'acide acétique se révèle inefficace pour obtenir une solubilisation totale de la phytine. En effet, même après l'ajout d'un volume de 65 ml d'une solution d'acide acétique à 0,3 N (soit un apport de 19,5 mmol en groupe carboxyle), la phytine n'est pas totalement dissoute comme l'atteste l'aspect trouble de la solution.

**Tableau 3 : Composition de la phytine riche en calcium commercialisée par Tsuno Rice Fine Chemicals Co., Ltd**

| Phytine commerciale extraite du son de riz riche en calcium commercialisée par Tsuno Rice Fine Chemicals Co., Ltd | |
|---|---|
| Pureté | 99,7 % |
| Teneur en phosphore | 20,7% |
| Teneur en magnésium | 4,0% |
| Teneur en calcium | 16,4 % |

| | |
|---|---|
| * les teneurs correspondent à des pourcentages massiques par rapport au poids total de la phytine commerciale. | |

### Exemple 2 : Procédé de préparation d'un complément alimentaire enrichi en phytine

### Etape 1 : Préparation d'une solution stabilisée de phytine

La phytine utilisée est la phytine riche en magnésium commercialisée par Tsuno Rice Fine Chemicals Co., Ltd et dont la composition est présentée au tableau 1 ci-dessus.

21,1 kg de phytine sont ajoutés progressivement dans 100 L d'eau purifiée sous agitation. Après homogénéisation, 25 L d'une solution aqueuse d'acide citrique (1,68 mol.L⁻¹) sont introduits progressivement dans le milieu réactionnel. Le milieu réactionnel est maintenu sous agitation pendant environ 45 minutes jusqu'à l'obtention d'une solution limpide de pH stabilisé.

La solution de phytine obtenue a un pH de 4,1, une teneur en phytine de 168,9 g/L et une concentration en acide citrique de 0,34 mol/L.

### Etape 2 : Ajout de composés à but Physiologique et obtention du complément alimentaire selon l'invention

A 125 L de la solution obtenue à l'étape 1 sont ajoutés, sous agitation, 0,68 kg de myo-inositol et 1,20 kg d'acide ascorbique. Le volume du mélange obtenu est ajusté à 150 L. Après homogénéisation, le mélange est filtré sur un filtre en nylon d'ouverture de maille de 50 µm.

Le complément alimentaire obtenu est une solution limpide de couleur jaune ambrée, de pH 4,0 et de composition suivante :
■ 140 g/L de phytine
■ 0,28 mol/L d'acide citrique soit une quantité de groupements fonctionnels carboxyles de 6 mmol par gramme de phytine
■ 4,5 g/L de myo-inositol et
■ 8 g/L d'acide ascorbique

Après l'étape de filtration, le complément alimentaire est conditionné en ampoules secables unidoses de 10 mL. Le complément alimentaire obtenu peut être conservé plusieurs mois à l'abri de la lumière, à température ambiante ou au réfrigérateur. En alternative, le complément alimentaire peut être conditionné en flacons de 200 ml munis d'un bouchon doseur. Pour ce type de conditionnement, le complément alimentaire doit de préférence comprendre un agent conservateur, être conservé au réfrigérateur et consommé de préférence dans les 20 jours après ouverture.

### Exemple 3 : Posologie du complément alimentaire selon l'invention

Dans le cas d'un traitement d'attaque afin de rétablir l'état physiologique du consommateur ou d'induire un effet physiologique particulier (par exemple la stimulation du système immunitaire), la posologie recommandée est une ampoule de 15 mL de complément alimentaire par jour ce qui correspond à une dose journalière de phytine de 2100 mg. La dose journalière de myo-inositol et d'acide ascorbique est de 120 mg et de 67,5 mg par jour, respectivement. Un tel régime est suivi sur 8 jours en moyenne et quelques semaines au plus.

Dans le cas d'un traitement de fond afin de maintenir l'état physiologique du patient et de prévenir l'apparition de certaines pathologies, la posologie recommandée est de 10 mL de complément alimentaire par jour, ce qui correspond à une dose de phytine de 1400 mg/jour, une dose d'acide ascorbique de 80 mg/jour et à une dose de myo-inositol de 45 mg/jour.

La prise du complément alimentaire peut être effectuée à tout moment de la journée. Néanmoins, il est recommandé de prendre le complément alimentaire selon la présente invention le matin à jeun, dilué dans de l'eau ou un jus de fruit.

## Revendications

1. Procédé de préparation d'un complément alimentaire liquide comprenant de la phytine **caractérisé en ce qu'**il comprend une étape (i) consistant à combiner une phytine, au moins un acide carboxylique alpha-hydroxylé et un solvant aqueux de manière à obtenir une solution aqueuse comprenant de la phytine dans un état solubilisé, de préférence à une teneur allant de 70g/L à 270 g/L, et ayant un pH allant de 3 à 5.

2. Procédé de préparation selon la revendication 1 **caractérisé en ce que** la quantité d'acide carboxylique alpha-hydroxylé utilisée à l'étape (i) correspond à un apport en groupe fonctionnel carboxyle allant de 3 mmol à 9 mmol par gramme de phytine.

3. Procédé de préparation d'un complément alimentaire selon l'une quelconque des revendications 1 à 2 **caractérisé en ce que** l'acide carboxylique alpha-hydroxylé est choisi parmi le groupe constitué par l'acide lactique, les diastéréoisomères de l'acide tartrique, l'acide glycolique, l'acide gluconique, l'acide glucuronique, l'acide citrique, l'acide malique et leurs mélanges.

4. Procédé selon l'une quelconque des revendications 1 à 3 **caractérisé en ce que** l'étape (i) comprend les étapes consistant à :
(a) préparer une suspension de phytine dans une solution aqueuse,
(b) ajouter à la suspension de l'étape (a) un acide carboxylique alpha-hydroxylé, de préférence sous la forme d'une solution aqueuse concentrée.

5. Procédé de préparation selon l'une quelconque des revendications 1 à 4 **caractérisé en ce que** la phytine est une phytine obtenue à partir de graines de céréales et présente un pourcentage massique en calcium de 0,05% à 20% et un pourcentage massique en magnésium de 3% à 18%, les pourcentages étant exprimés par rapport au poids total de phytine.

6. Procédé de préparation d'un complément alimentaire selon l'une quelconque des revendications 1 à 5 **caractérisé en ce qu'**il comprend l'étape (ii) consistant à ajouter à la solution obtenue à l'étape (i) au moins un composé supplémentaire choisi parmi le groupe constitué par les minéraux, les vitamines et leurs précurseurs, les acides aminés et leurs dérivés, les oligopeptides, les extraits de plantes, les acides gras, les hormones, les flavonoïdes, le myo-inositol, et leurs mélanges.

7. Procédé de préparation d'un complément alimentaire selon la revendication 6 **caractérisé en ce que** l'on ajoute à l'étape (ii) :
■ de l'acide ascorbique ou un de ses sels de telle sorte à ce que la teneur finale en acide ascorbique dans le complément alimentaire soit comprise entre 6 g/L et 12 g/L, et
■ du myo-inositol de telle sorte à ce que la teneur finale en myo-inositol dans le complément alimentaire soit comprise entre 3 g/L et 6 g/L.

8. Procédé de préparation d'un complément alimentaire liquide selon l'une quelconque des revendications 1 à 6 **caractérisé en ce qu'**il comprend une étape de filtration.

9. Complément alimentaire liquide comprenant de la phytine **caractérisé en ce que**
(i) il s'agit d'une solution aqueuse ayant un pH allant de 3 à 7,5, de préférence de 3 à 5,
(ii) il comprend de la phytine sous forme solubilisée en présence d'au moins un acide carboxylique alpha-hydroxylé, ladite phytine étant présente à une teneur allant de 70 g/L à 270 g/L, étant entendu que ladite teneur en phytine est exprimée par rapport au volume total dudit complément alimentaire et
(iii) facultativement, au moins un composé supplémentaire choisi parmi le groupe constitué par les minéraux, les vitamines et leurs précurseurs, les acides aminés et leurs dérivés, les oligopeptides, les extraits de plantes, les acides gras, les hormones, les flavonoïdes, le myo-inositol, et leurs mélanges.

10. Complément alimentaire selon la revendication 9 **caractérisé en ce que** la teneur en acide carboxylique alpha-hydroxylé correspond à une quantité de groupe fonctionnel carboxyle allant de 3 mmol à 9 mmol par gramme de phytine.

11. Complément alimentaire selon l'une quelconque des revendications 9 à 10 **caractérisé en ce qu'**il comprend :
■ du myo-inositol à une teneur comprise entre 3 g/L et 6 g/L, et
■ de l'acide ascorbique ou un de ses sels à une teneur comprise entre 6 g/L et 12 g/L.

12. Solution aqueuse de phytine **caractérisée en ce que** :
■ elle comprend au moins un acide carboxylique alpha-hydroxylé,
■ elle comprend de la phytine sous forme solubilisée à une teneur allant de 70 g/L à 270 g/L et
■ elle a un pH allant de 3 à 5

13. Solution aqueuse de phytine selon la revendication 12 **caractérisée en ce que** la quantité d'acide carboxylique alpha-hydroxylé correspond à un apport en groupe fonctionnel carboxyle de 3 mmol à 9 mmol par gramme de phytine.

14. Utilisation d'une solution aqueuse de phytine telle que définie dans la revendication 12 ou la revendication 13 pour la préparation d'un complément alimentaire ou pour la préparation d'une composition cosmétique.

## Claims

1. A method for preparing a liquid nutritional supplement comprising phytin, **characterized in that** it comprises a step (i) consisting in combining a phytin, at least one alpha-hydroxylated carboxylic acid and an aqueous solvent so as to obtain an aqueous solution comprising phytin in solubilized state, preferably in a concentration ranging from 70g/L to 270 g/L, and with a pH value of from 3 to 5.

2. A preparation method according to claim 1, **characterized in that** the amount of alpha-hydroxylated carboxylic acid used in step (i) corresponds to a carboxyl functional group proportion ranging from 3 mmol to 9 mmol per gram of phytin.

3. A method for preparing a nutritional supplement according to any one of claims 1 to 2, **characterized in that** the alpha-hydroxylated carboxylic acid is selected in the group consisting of lactic acid, diastereoisomers of tartaric acid, glycolic acid, gluconic acid, glucuronic acid, citric acid, malic acid and their combinations.

4. A method according to any one of claims 1 to 3, **characterized in that** (i) comprises the steps consisting in:
(a) preparing a phytine suspension in aqueous solution,
(b) adding to the suspension of step (a) an alpha-hydroxylated carboxylic acid, preferably as an aqueous concentrated solution

5. A preparation method according to any one of claims 1 to 4, **characterized in that** phytin is a phytine obtained from cereal seeds and has a calcium content, in weight percent, of from 0.05% to 20% and a magnesium content, in weight percent, of from 3% to 18%, the percentages being expressed relative to the phytine total weight.

6. A method for preparing a nutritional supplement according to any one of claims 1 to 5, **characterized in that** it comprises step (it) consisting in adding to the solution obtained in step (i) at least on additional compound selected in the group consisting of minerals, vitamins and precursors thereof, amino acids and derivatives thereof, oligopeptides, plant extracts, fatty acids, hormones, flavonoids, myo-inositol, and their combinations.

7. A method for preparing a nutritional supplement according to claim 6, **characterized in that** are added in step (ii):
■ ascorbic acid, or a salt thereof, so that the end concentration in ascorbic acid in the nutritional supplement be comprised between 6 g/L and 12 g/L, and
■ myo-inositol, so that the end concentration in myo-inositol in the nutritional supplement be comprised between 3 /L and 6 g/L.

8. A method for preparing a liquid nutritional supplement according to any one of claims 1 to 6, **characterized in that** it comprises a step of filtration.

9. A liquid nutritional supplement comprising phytin, **characterized in that**
(i) it is an aqueous solution with a pH value of from 3 to 7.5. preferably of from 3 to 5.
(ii) it comprises phytin in a solubilized form, together with at least one alpha-hydroxylated carboxylic acid, said phytin being present in a concentration ranging from 70 g/L to 270 g/L, the phytin content being expressed relative to the nutritional supplement total volume and
(iii) optionally, at least one additional compound selected in the group consisting of minerals, vitamins and precursors thereof, amino acids and derivatives thereof, oligopeptides, plant extracts, fatty acids, hormones, flavonoids, myo-inositol. and their combinations.

10. A nutritional supplement according to claim 9, **characterized in that** the content of alpha-hydroxylated carboxylic acid corresponds to a carboxyl functional group amount ranging from 3 mmol to 9 mmol per gram of phytin.

11. A nutritional supplement according to any one of claims 9 to 11, **characterized in that** it comprises:
■ myo-inositol in a concentration ranging from 3 g/L to 6 g/L, and
■ ascorbic acid, or a salt thereof, in a concentration ranging from 6 g/L to 12 g/L.

12. A phytin aqueous solution **characterized in that**:
■ it comprises at least one alpha-hydroxylated carboxylic acid,
■ it comprises phytin, in a solubilized form, in a concentration ranging from 70 g/L to 270 g/L and
■ its pH value does range from 3 to 5.

13. A phytin aqueous solution according to claim 12, **characterized in that** the amount of alpha-hydroxylated carboxylic acid corresponds to a carboxyl functional group proportion ranging from 3 mmol to 9 mmol per gram of phytin.

14. Use of a phytin aqueous solution as defined according to claim 12 or 13, for the preparation of a nutritional supplement or for the preparation of a cosmetic composition.

## Patentansprüche

1. Verfahren zur Herstellung einer flüssigen Nahrungsergänzung, die Phytin umfasst, **dadurch gekennzeichnet, dass** es einen Schritt (i) des Kombinierens eines Phytins, wenigstens einer α-Hydroxycarbonsäure und eines wässrigen Lösungsmittels in einer solchen Weise umfasst, dass man eine wässrige Lösung erhält, die Phytin in einem gelösten Zustand, vorzugsweise in einem Gehalt von 70 g/l bis 270 g/l, umfasst und einen pH-Wert von 3 bis 5 aufweist.

2. Herstellungsverfahren gemäß Anspruch 1, **dadurch gekennzeichnet, dass** die in Schritt (i) verwendete Menge an α-Hydroxycarbonsäure einem Zuwachs an funktionellen Carboxygruppen von 3 mmol bis 9 mmol pro Gramm Phytin entspricht.

3. Verfahren zur Herstellung einer Nahrungsergänzung gemäß einem der Ansprüche 1 bis 2, **dadurch gekennzeichnet, dass** die α-Hydroxycarbonsäure aus der Gruppe ausgewählt ist, die aus Milchsäure, Diastereomeren der Weinsäure, Glycolsäure, Gluconsäure, Glucuronsäure, Zitronensäure, Äpfelsäure und Gemischen davon besteht.

4. Verfahren gemäß einem der Ansprüche 1 bis 3, **dadurch gekennzeichnet, dass** Schritt (i) die folgenden Schritte umfasst:
(a) Herstellen einer Phytinsuspension in einer wässrigen Lösung ;
(b) Hinzufügen einer α-Hydroxycarbonsäure, vorzugsweise in Form einer konzentrierten wässrigen Lösung, zur Suspension von Schritt (a).

5. Herstellungsverfahren gemäß einem der Ansprüche 1 bis 4, **dadurch gekennzeichnet, dass** das Phytin ein Phytin ist, das ausgehend von Getreidekörnern erhalten wird und einen Calciumgehalt von 0,05 bis 20 Gew.-% und einen Magnesiumgehalt von 3 bis 18 Gew.-% aufweist, wobei die Prozentwerte in Bezug auf das Gesamtgewicht des Phytins ausgedrückt sind.

6. Verfahren zur Herstellung einer Nahrungsergänzung gemäß einem der Ansprüche 1 bis 5, **dadurch gekennzeichnet, dass** es den Schritt (ii) des Hinzufügens wenigstens einer ergänzenden Verbindung, die aus der Gruppe ausgewählt ist, die aus Mineralstoffen, Vitaminen und deren Vorläufern, Aminosäuren und ihren Derivaten, Oligopeptiden, Pflanzenextrakten, Fettsäuren, Hormonen, Flavonoiden, myo-Inosit und Gemischen davon besteht, zu der in Schritt (i) erhaltenen Lösung umfasst.

7. Verfahren zur Herstellung einer Nahrungsergänzung gemäß Anspruch 6, **dadurch gekennzeichnet, dass** in Schritt (ii) Folgendes hinzugefügt wird:
- Ascorbinsäure oder eines ihrer Salze in einer solchen Menge, dass der Endgehalt an Ascorbinsäure in der Nahrungsergänzung im Bereich von 6 g/l bis 12 g/l liegt; und
- myo-Inosit in einer solchen Menge, dass der Endgehalt an myo-Inosit in der Nahrungsergänzung im Bereich von 3 g/l bis 6 g/l liegt.

8. Verfahren zur Herstellung einer flüssigen Nahrungsergänzung gemäß einem der Ansprüche 1 bis 6, **dadurch gekennzeichnet, dass** es einen Filtrationsschritt umfasst.

9. Flüssige Nahrungsergänzung, die Phytin umfasst, durch gekennzeichnet, dass
(i) es sich um eine wässrige Lösung mit einem pH-Wert von 3 bis 7,5, vorzugsweise 3 bis 5 handelt;
(ii) sie das Phytin in gelöster Form in Gegenwart wenigstens einer α-Hydroxycarbonsäure umfasst, wobei das Phytin in einem Gehalt von 70 g/l bis 270 g/l vorhanden ist, wobei der Gehalt an Phytin auf das Gesamtvolumen der Nahrungsergänzung bezogen ist; und
(iii) gegebenenfalls wenigstens eine ergänzende Verbindung, die aus der Gruppe ausgewählt ist, die aus Mineralstoffen, Vitaminen und deren Vorläufern, Aminosäuren und ihren Derivaten, Oligopeptiden, flanzenextrakten, Fettsäuren, Hormonen, Flavonoiden, myo-Inosit und Gemischen davon besteht, umfasst.

10. Nahrungsergänzung gemäß Anspruch 9, **dadurch gekennzeichnet, dass** der Gehalt an α-Hydroxycarbonsäure einer Menge an funktionellen Carboxygruppen von 3 mmol bis 9 mmol pro Gramm Phytin entspricht.

11. Nahrungsergänzung gemäß einem der Ansprüche 9 bis 10, **dadurch gekennzeichnet, dass** sie Folgendes umfasst:
- myo-Inosit in einem Gehalt im Bereich von 3 g/l bis 6 g/l; und
- Ascorbinsäure oder eines ihrer Salze in einem Gehalt im Bereich von 6 g/l bis 12 g/l.

12. Wässrige Phytinlösung, **dadurch gekennzeichnet, dass**:
- sie wenigstens eine α-Hydroxycarbonsäure umfasst;
- sie Phytin in gelöster Form in einem Gehalt im Bereich von 70 g/l bis 270 g/l umfasst; und
- sie einen pH-Wert im Bereich von 3 bis 5 aufweist.

13. Wässrige Phytinlösung gemäß Anspruch 12, **dadurch gekennzeichnet, dass** die Menge der α-Hydroxycarbonsäure einem Zuwachs an funktionellen Carboxygruppen von 3 mmol bis 9 mmol pro Gramm Phytin entspricht.

14. Verwendung einer wässrigen Phytinlösung, wie sie in Anspruch 12 oder 13 definiert ist, zur Herstellung einer Nahrungsergänzung oder zur Herstellun einer kosmetischen Zusammensetzung.
